# EUROPEAN PATENT SPECIFICATION

(11) **EP 3 991 658 B1**
(45) Date of publication and mention of the grant of the patent: **14.02.2024**
(21) Application number: 21206025.5
(22) Date of filing: 02.11.2021
(51) Int. Cl.: A61B 5/327

(54) **RECORDING APPARATUS NOISE REDUCTION**
RAUSCHVERMINDERUNG FÜR AUFZEICHNUNGSVORRICHTUNG
RÉDUCTION DE BRUIT D'APPAREIL D'ENREGISTREMENT

(30) Priority: 03.11.2020 US 202063108998 P; 21.06.2021 US 202117352732
(43) Date of publication of application: 04.05.2022
(73) Proprietor: Biosense Webster (Israel) Ltd, Yokneam, 2066717 (IL)
(72) Inventor: GLINER, Vadim, 2066717 Yokneam (IL)
(74) Representative: Carpmaels & Ransford LLP

(56) References cited:
- EP-A1- 3 960 074
- CN-A- 110 141 215

## Description

### RELATED APPLICATION INFORMATION

The present application claims benefit of US Provisional Patent Application 63/108,998, filed 3 November 2020.

### FIELD OF THE INVENTION

The present invention relates to medical equipment, and in particular, but not exclusively to, reducing noise in electrogram signals.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied between the catheter electrode(s) of the ablating catheter and an indifferent electrode (which may be one of the catheter electrodes), and current flows through the media between the electrodes, i.e., blood and tissue. The distribution of current may depend on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. In some applications, irreversible electroporation may be performed to ablate the tissue.

EP3960074 A1 falls under Art. 54(3) EPC and discloses a method and system for removing noise from cardiac signals. A method includes receiving first cardiac signals captured by at least one first sensing electrode in contact with tissue of a first living subject, injecting the received first cardiac signals into a length of wire electrically connected at both ends to a processing circuitry, which outputs respective noise-added cardiac signals responsively to noise acquired in the wire, training an artificial neural network to remove noise from cardiac signals responsively to the received first cardiac signals and the respective noise-added cardiac signals, receiving second cardiac signals captured by at least one second sensing electrode in contact with tissue of a second living subject, and applying the trained artificial neural network to the second cardiac signals to yield noise-reduced cardiac signals.

CN 110 141 215 A discloses a noise reduction auto-encoder training method, and an electrocardiosignal noise reduction method, related device and equipment. Artificial intelligence is used to conduct noise reduction of electrocardiosignals and the method can be applied in fields of intelligent detection electrocardiograms, etc. The method extracts obvious characteristic noise-to-be-reduced standard electrocardiosignals of R peak positions, R-R intervals, etc. of noise-to-be-reduced electrocardiosignals from the noise-to-be-reduced electrocardiosignals, a target noise reduction auto-encoder conducts noise reduction of noise-to-be-reduced left electrocardiosignals after the noise-to-be-reduced standard electrocardiosignals of the noise-to-be-reduced electrocardiosignals are removed, the target noise reduction auto-encoder is avoided to conduct a noise reduction treatment of obvious characteristics in the noise-to-be-reduced electrocardiosignals, so that the noise-to-be-reduced standard electrocardiosignals and the noise-to-be-reduced left electrocardiosignals are superimposed to obtain the noise-reduced electrocardiosignals, which can better preserve the R peak positions in the noise-to-be-reduced electrocardiosignals and reduce distortion of the noise-reduced electrocardiosignals after the noise reduction.

### SUMMARY

There is provided in accordance with the invention a medical system as claimed hereinafter

### BRIEF DESCRIPTION OF THE DRAWINGS

The present invention will be understood from the following detailed description, taken in conjunction with the drawings in which:
Fig. 1 is a pictorial illustration of a system for performing catheterization procedures on a heart, constructed and operative in accordance with an exemplary embodiment of the present invention;
Fig. 2 is a perspective view of a catheter for use with the system of Fig. 1;
Fig. 3 is a detailed schematic view of an electrode assembly for use with the system of Fig. 1;
Fig. 4 is a more detailed view of processing circuitry in the system of Fig. 1;
Fig. 5 is a flowchart including steps in a method of operation of the system of Fig. 1;
Fig. 6 is a schematic view of an artificial neural network for use with the system of Fig. 1;
Fig. 7 is a schematic view illustrating training of the artificial neural network of Fig. 6;
Fig. 8 is a flowchart including sub-steps in a step of the method of Fig. 5;
Fig. 9 is a schematic view illustrating processing of a captured signal being processed by the trained artificial neural network; and
Fig. 10 is a flowchart including steps in a method to process the captured signal of Fig. 9 using the trained artificial neural network.

### DESCRIPTION OF EXAMPLE EMBODIMENTS

### OVERVIEW

As mentioned previously, during a cardiac electrophysiological (EP) investigative or ablation procedure, the leads between a patient and the electrical system used for the procedure, such as the Carto^{®} system (Biosense Webster, Inc., of Irvine CA) and/or an external recording apparatus, may pick up noise. Even where the noise is from known equipment, it may be difficult or even impossible to remove the equipment. For example, the Carto system may use an external uninterruptible power supply (UPS) which cannot be removed, but which generates electrical noise. The Carto system typically uses filtering techniques to clean the signal of the noise. However, the signal recorded by the external recording apparatus may be very noisy, which may be a significant problem due to the very small voltages associated with electrophysiological signals. Additionally, the external recording apparatus is commonly an analogue device without noise filtering capabilities and therefore cannot filter the noise from the received cardiac signals.

Embodiments of the present invention reduce the problems associated with electrical noise pick-up in a cable between a catheter (and/or body surface electrodes) and the recording apparatus by routing the cardiac signals captured by the catheter (and/or body surface electrodes) via processing circuitry (for example, which is part of the Carto system). The processing circuitry compensates for expected noise pickup prior to outputting the cardiac signals to the external recording apparatus over a recording apparatus cable. The compensation is performed using an artificial neural network (ANN), which is trained to at least partially compensate for the electrical noise, which is not yet in the cardiac signals but will be added to the cardiac signals in the cable from the processing circuitry to the recording apparatus.

The ANN is trained as follows. The processing circuitry receives cardiac signal segments (which may be segments of the same signal) responsively to electrical activity sensed by one or more sensing electrodes (e.g., of a catheter and/or body surface electrodes) in contact with tissue of a living subject. The processing circuitry is electrically connected to the recording apparatus via one end of a recording apparatus cable, which is generally unshielded and therefore signals is this cable pick up surrounding electrical noise. The other end of the recording apparatus cable may also be electrically connected to a shielded cable which extends back to the processing circuitry. The term "shielded cable", as used in the specification or claims, is defined as an electrical cable of one or more insulated conductors enclosed by a common conductive layer, which may be composed of braided strands of copper (or other metal, such as aluminum), a non-braided spiral winding of copper tape, or a layer of conducting polymer.

The processing circuitry injects the received cardiac signal segments into the recording apparatus cable where the cardiac signal segments pick up noise so that the recording apparatus cable outputs noise-added cardiac signal segments. The noise-added cardiac signal segments may then pass through the shielded cable back to the processing circuitry.

The processing circuitry trains the artificial neural network to at least partially compensate for electrical noise that will be added to cardiac signals in the cable responsively to the received cardiac signal segments and the corresponding noise-added cardiac signal segments. In some embodiments, the processing circuitry inputs the noise-added cardiac signal segments into the artificial neural network and iteratively adjusts parameters (e.g., weights) of the artificial neural network to reduce a difference between an output of the artificial neural network and the received cardiac signal segments.

### SYSTEM DESCRIPTION

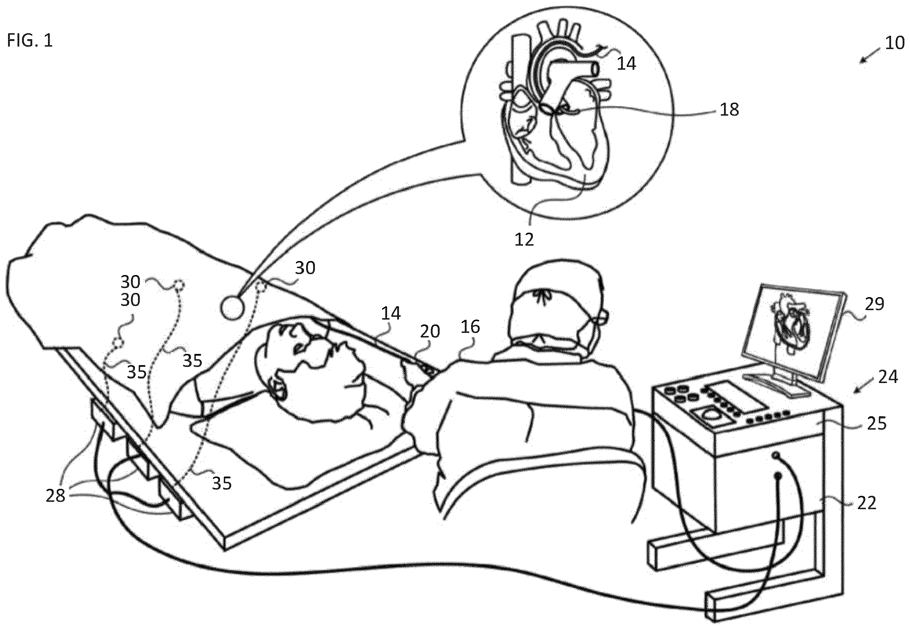

Reference is now made to Fig. 1, which is a pictorial illustration of a medical system 10 for performing catheterization procedures on a heart 12, constructed and operative in accordance with an embodiment of the present invention. The medical system 10 may be configured to evaluate electrical activity and perform ablative procedures on the heart 12 of a living subject. The system 10 comprises a catheter 14, which is percutaneously inserted by an operator 16 through the patient's vascular system into a chamber or vascular structure of the heart 12. The operator 16, who is typically a physician, brings the catheter's distal end 18 into contact with the heart wall, for example, at an ablation target site. Electrical activation maps may be prepared, according to the methods disclosed in U.S. Patent Nos. 6,226,542, 6,301,496, and 6,892,091. One commercial product embodying elements of the system 10 is available as the CARTOR 3 System, available from Biosense Webster, Inc., Irvine, CA. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at the distal end 18, which apply the radiofrequency energy to the myocardium. The energy is absorbed in the tissue, heating it to a point at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

The catheter 14 typically comprises a handle 20, having suitable controls on the handle to enable the operator 16 to steer, position and orient the distal end 18 of the catheter 14 as desired for the ablation. To aid the operator 16, a distal portion of the catheter 14 contains position sensors (not shown) that provide signals to processing circuitry 22, located in a console 24. The processing circuitry 22 may fulfill several processing functions as described below.

Wire connections 35 may link the console 24 with body surface electrodes 30 and other components of a positioning sub-system for measuring location and orientation coordinates of the catheter 14. The processing circuitry 22 or another processor (not shown) may be an element of the positioning subsystem. Catheter electrodes (not shown) and the body surface electrodes 30 may be used to measure tissue impedance at the ablation site as taught in U.S. Patent No. 7,536,218. Temperature sensors (not shown), typically a thermocouple or thermistor, may be mounted on ablation surfaces on the distal portion of the catheter 14 as described below.

The console 24 typically contains one or more ablation power generators 25. The catheter 14 may be adapted to conduct ablative energy to the heart using any known ablation technique, e.g., radiofrequency energy, ultra-sound energy, irreversible electroporation and laser-produced light energy. Such methods are disclosed in U.S. Patent Nos. 6,814,733, 6,997,924, and 7,156,816.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent Nos. 7,756,576, and 7,536,218.

As noted above, the catheter 14 is coupled to the console 24, which enables the operator 16 to observe and regulate the functions of the catheter 14. Console 24 includes the processing circuitry 22, generally a computer with appropriate signal processing circuits. The processing circuitry 22 is coupled to drive a display 29 (e.g., a monitor). The signal processing circuits typically receive, amplify, filter and digitize signals from the catheter 14, including signals generated by sensors such as electrical, temperature and contact force sensors, and a plurality of location sensing electrodes (not shown) located distally in the catheter 14. The digitized signals are used by the console 24 and the positioning system to compute the position and orientation of the catheter 14, and to analyze the electrical signals from the electrodes.

In order to generate electroanatomic maps, the processing circuitry 22 typically comprises a mapping module including an electroanatomic map generator, an image registration program, an image or data analysis program and a graphical user interface configured to present graphical information on the display 29.

Typically, the system 10 includes other elements, which are not shown in the figures for the sake of simplicity. For example, the system 10 may include an electrocardiogram (ECG) monitor, coupled to receive signals from one or more of the body surface electrodes 30, in order to provide an ECG synchronization signal to the console 24. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site may be provided. The system 10 may receive image data from an external imaging modality, such as an MRI unit or the like and includes image processors that can be incorporated in or invoked by the processing circuitry 22 for generating and displaying images.

In practice, some or all of the functions of the processing circuitry 22 may be combined in a single physical component or, alternatively, implemented using multiple physical components. These physical components may comprise hard-wired or programmable devices, or a combination of the two. In some embodiments, at least some of the functions of the processing circuitry 22 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

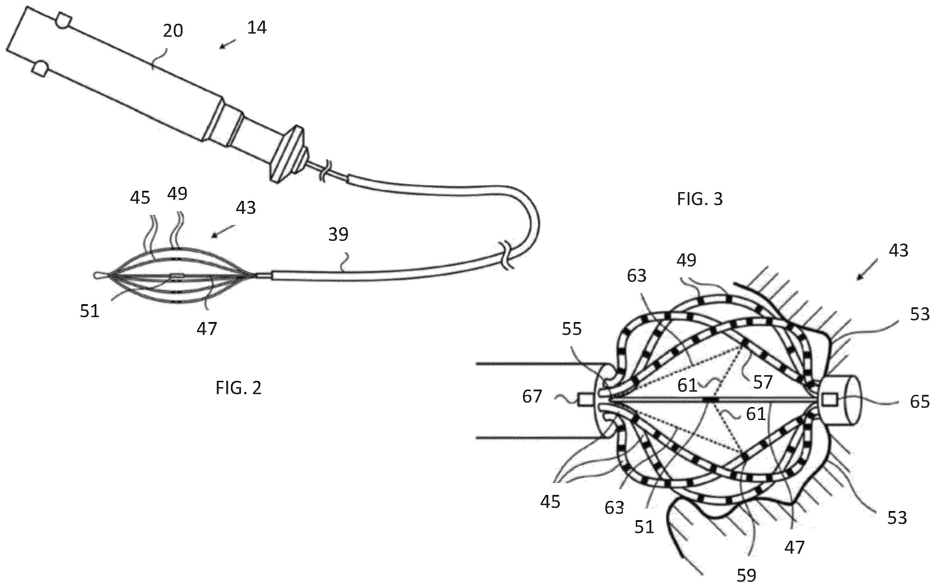

Reference is now made to Fig. 2, which is a perspective view of the catheter 14 for use with the system 10 of Fig. 1.

The catheter 14 comprises an elongated shaft 39 having proximal and distal ends, the control handle 20 at the proximal end of the catheter body, and an expandable distal end basket assembly 43 mounted at the distal end of the shaft 39.

The shaft 39 comprises an elongated tubular construction having a single, axial or central lumen (not shown), but can optionally have multiple lumens if desired. The shaft 39 is flexible, i.e., bendable, but substantially non-compressible along its length. The shaft 39 can be of any suitable construction and made of any suitable material. In some embodiments, the elongated shaft 39 comprises an outer wall made of polyurethane or polyether block amide. The outer wall comprises an imbedded braided mesh of stainless steel or the like to increase torsional stiffness of the shaft 39 so that, when the control handle 20 is rotated, the distal end of the shaft 39 rotates in a corresponding manner.

The outer diameter of the shaft 39 is not critical, but may be in the range of about 2 to 5 mm. Likewise, the thickness of the outer wall is not critical, but is generally thin enough so that the central lumen can accommodate any one or more of the following: a puller wire, lead wires, sensor cables and any other wires, cables or tubes. If desired, the inner surface of the outer wall is lined with a stiffening tube (not shown) to provide improved torsional stability. An example of a catheter body construction suitable for use in connection with the present invention is described and depicted in U.S. Patent No. 6,064,905.

The assembly 43 is mounted to the distal end of the shaft 39. As shown in Fig. 2, the basket assembly 43 comprises five splines 45 or arms mounted, generally evenly-spaced, around a contraction wire 47, which is connected to the distal extremity of the assembly 43, and which contracts, retracts and expands the assembly 43 when a tractive or a pushing force is applied longitudinally to the contraction wire 47 as the case may be. The contraction wire 47 forms a longitudinal axis of symmetry for the assembly 43. The splines 45 are all attached, directly or indirectly, to the contraction wire 47 at their distal ends, and to the shaft 39 at their proximal ends. When the contraction wire 47 is moved longitudinally to expand and contract the assembly 43, in the expanded position the splines 45 are bowed outwardly and in the contracted position the splines 45 are generally straight. As will be recognized by one skilled in the art, the number of splines 45 can vary as desired depending on the particular application, so that the assembly 43 has at least two splines, generally at least three splines, and as many as ten or more splines. The expandable distal end basket assembly 43 is not limited to the depicted configuration, but can include other designs, such as spherical or egg-shaped designs, that include a plurality of expandable arms connected, directly or indirectly, at their proximal and distal ends. In other embodiments, the basket assembly may be replaced by any suitable distal end assembly, for example, a balloon assembly, a focal catheter assembly, a flat grid assembly, a multiple spline assembly, or a focal catheter assembly.

The assembly 43 includes at least one sensing electrode 49 disposed thereon. In some embodiments, each of the splines 45 may comprise a flexible wire with a non-conductive covering on which one or more of the sensing electrodes 49 (e.g., ring spline electrodes) are mounted. The electrodes 49 are termed as "sensing electrodes" for the sake of convenience, but may also be used to perform ablation. In some embodiments, the flexible wires each comprise a flat nitinol wire and the non-conductive coverings each comprise a biocompatible plastic tubing, such as polyurethane or polyimide tubing. Alternatively, the splines 45 can be designed without the internal flexible wire if a sufficiently rigid nonconductive material is used for the non-conductive covering to permit expansion of the assembly 43, so long as the spline has an outer surface that is non-conductive over at least a part of its surface for mounting of the sensing electrodes 49. In some embodiments, the splines may be formed from flexible polymer strip circuits with electrodes 49 being disposed on an outer surface of each of the flexible polymer strip circuits.

Each of the sensing electrodes 49 on the splines 45 is electrically connected to an appropriate mapping or monitoring system and/or source of ablation energy by means of an electrode lead wire (not shown). The electrode lead wires extend through the control handle 20, through a lumen in the shaft 39, into the non-conductive covering of corresponding splines 45, and attach to their corresponding sensing electrodes 49 by any suitable method. The catheter 14 optionally includes a far-field electrode 51, e.g., a cylindrical electrode, disposed on the contraction wire 47. The far-field electrode 51 is disposed in the expandable distal end basket assembly 43 to prevent the far-field electrode 51 from contacting the tissue of the cardiac chamber of the heart 12. The function of the far-field electrode 51 is described below with reference to Fig. 3. Additional details of the catheter 14 are described in the above-referenced U.S. Patent No. 6,748,255. The catheter 14 typically has multiple sensing electrodes 49 arranged on multiple flexible splines of the basket assembly 43. The catheter 14 is configured to be inserted into a cardiac chamber of the heart 12 (Fig. 1) of the living subject in a collapsed form, where the splines 45 are relatively close together. One or more of the sensing electrodes 49 are configured to make contact with tissue of the living subject. Once in the heart 12, the splines 45 may be formed into their expanded basket shape by the contraction wire 47, which holds distal ends of the splines 45, and pulls the distal ends of the splines 45 in a proximal direction.

Reference is now made to Fig. 3, which is a detailed schematic view of the expandable distal end basket assembly 43 of Fig. 2. In expanded form of the assembly 43 at least a portion of the sensing electrodes 49 of the splines 45 contact endocardial surface 53 of the heart 12 and acquire signals corresponding to electropotentials generated at their points of contact with the surface. However, since the sensing electrodes 49 are in a conductive medium (the blood), in addition to the electropotentials from the points of contact, the acquired signals also include far-field components from other regions of the heart 12.

The far-field components constitute an interfering signal on the endocardial surface electropotentials. To counteract the interference, some embodiments position the far-field electrode 51 on the contraction wire 47. In the expanded configuration of the assembly 43, the far-field electrode 51 is located on the contraction wire 47 so as to be approximately equidistant from all corresponding sensing electrodes 49, i.e., sensing electrodes 49 that are equidistant from a fixed reference point on the long axis of the catheter, such as reference point 55 at the proximal end of the assembly 43, and is prevented from contacting the surface of the heart by the splines 45. For example, electrode 57, 59 are equidistant from reference point 55, and are also equidistant from the far-field electrode 51, as indicated by broken line 61, 63, respectively. When the far-field electrode 51 is at least 0.5 cm removed from the sensing electrodes 49 in the expanded configuration of the assembly 43 it acquires a far-field signal, but not a near-field signal from the endocardial surface 53. However, the signals e(t) acquired by the sensing electrodes 49 may have both a far-field and a surface (near-field) component. The far-field component signal x(t) acquired by the far-field electrode 51 may be removed from the signals e(t) acquired by the sensing electrodes 49 so as to counteract the interference suffered by these electrodes, i.e., by subtraction of the signals: e(t) - x(t). Additionally, or alternatively, removal of the far-field component may be accomplished using any suitable method, such as the algorithms described in US Patent Publication No. 2016/0175023, or US Patent No. 9,554,718. In some embodiments, the far-field components of the signals captured by the sensing electrodes 49 are not removed.

In some embodiments, the catheter 14 is provided with a distal location sensor 65 mounted at or near the position where the distal ends of the spines are connected, and a proximal location sensor 67 mounted at or near the proximal end of the assembly 43, whereby, in use, the co-ordinates of the location sensor 65 relative to those of the location sensor 67 can be determined and taken together with known information pertaining to the curvature of the splines 45 to find the positions of each of the sensing electrodes 49.

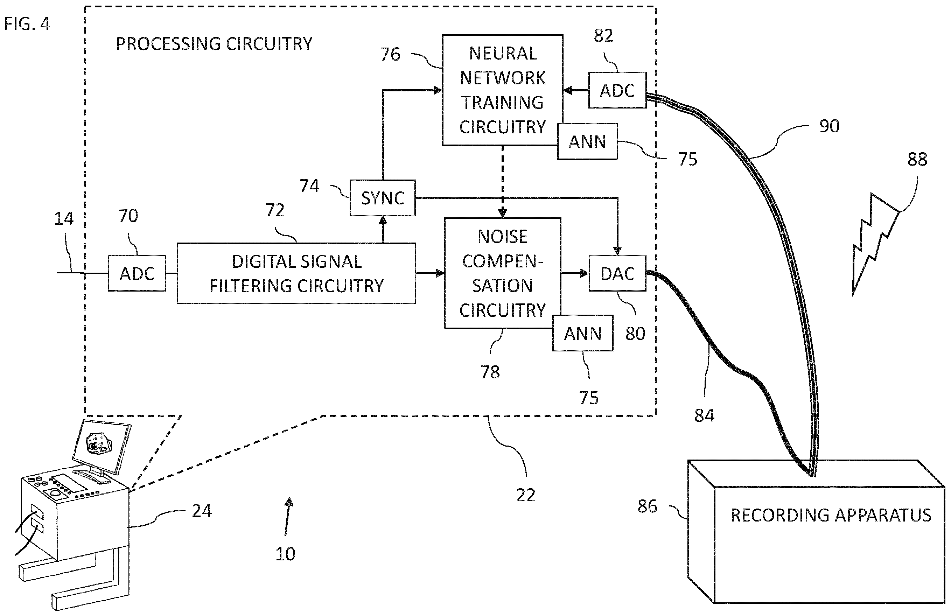

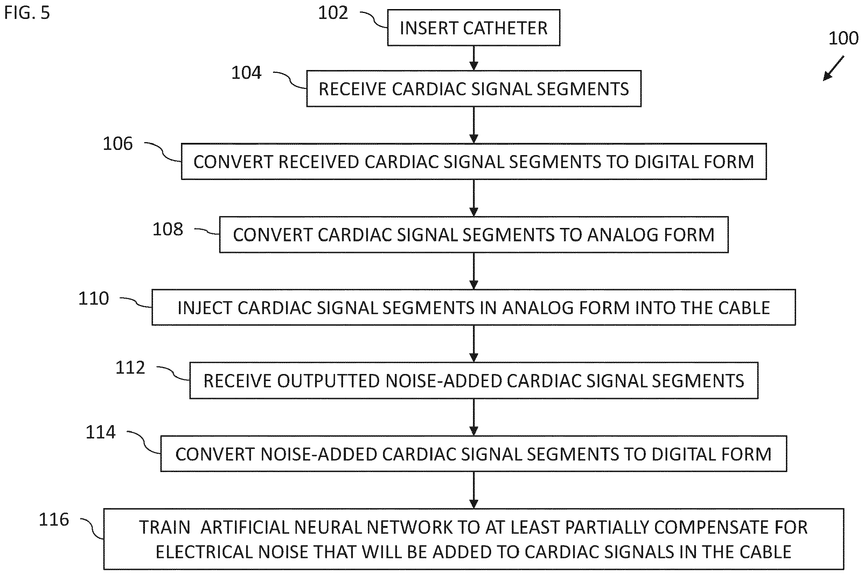

Reference is now made to Figs. 4 and 5. Fig. 4 is a more detailed view of the processing circuitry 22 in the system 10 of Fig. 1. Fig. 5 is a flowchart 100 including steps in a method of operation of the system 10 of Fig. 1.

The processing circuitry 22 includes an analog-to-digital converter 70, digital signal filtering circuitry 72, synchronization circuitry 74, neural network training circuitry 76, noise-compensation circuitry 78, a digital-to-analog converter 80, and an analog-to-digital converter 82. In some embodiments, the analog-to-digital converter 70 and analog-to-digital converter 82 may be implemented in a single multichannel analog-to-digital converter.

The synchronization circuitry 74, neural network training circuitry 76, and/or noise-compensation circuitry 78, may be implemented using software running on a processor and/or using hard-wired processing circuits. The software may be downloaded to the computer(s) or processor(s) in electronic form, over a network, for example. Alternatively, or additionally, the software may be provided on non-transitory tangible media, such as optical, magnetic, or electronic storage media.

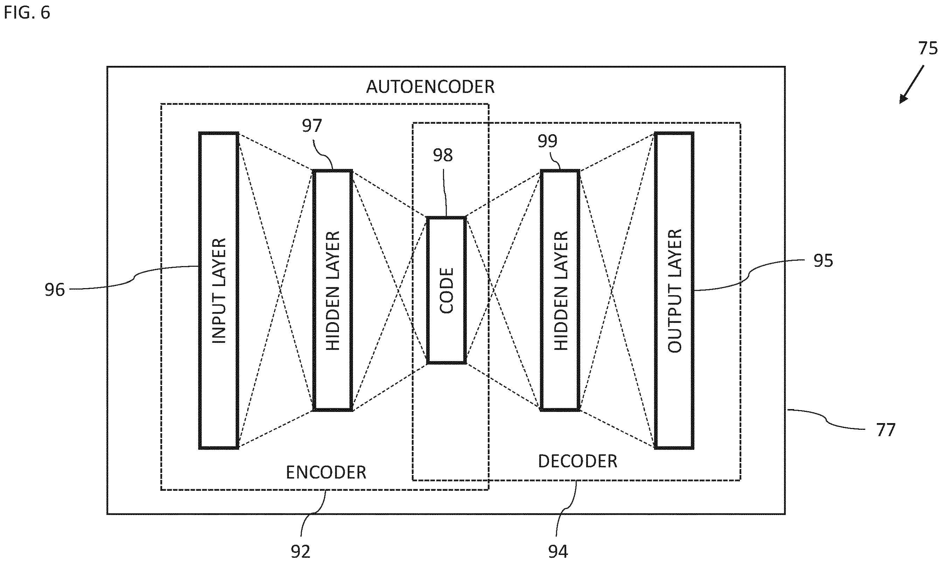

The neural network training circuitry 76 is configured to train an artificial neural network 75 as described in more detail below with reference to Figs. 6-8. The artificial neural network 75 may include an autoencoder 77 described in more detail with reference to Fig. 6.

The medical system 10 includes a recording apparatus cable 84 extending to a recording apparatus 86. The medical system 10 also includes a shielded cable 90.

One end of the recording apparatus cable 84 is connected to the processing circuitry 22 via the digital-to-analog converter 80. The other end of the recording apparatus cable 84 is connected to the recording apparatus 86. The recording apparatus cable 84 includes individual insulated wires which are generally not shielded, or not sufficiently shielded, and therefore pick up electrical noise 88 from the environment of the surrounding electrophysiological (EP) laboratory.

The recording apparatus cable 84 generally includes respective insulated wires to carry respective signals captured by respective ones of the sensing electrodes 49 (Fig. 3), and optionally the body surface electrodes 30 (Fig. 1), from the processing circuitry 22 to the recording apparatus 86. The recording apparatus cable 84 may also include one or more calibration wires to carry respective calibration signal(s) from the digital-to-analog converter 80 to the end of the recording apparatus cable 84 adjacent to the recording apparatus 86. The calibration wire(s) of the recording apparatus cable 84 are electrically connected (at the end of the recording apparatus cable 84 connected to the recording apparatus 86) to wires of the shielded cable 90 (at the end of the shielded cable 90 closest to the recording apparatus 86), which extends back to the processing circuitry 22 via the analog-to-digital converter 82.

The artificial neural network 75 is trained based on data captured from: a catheter such as the catheter 14 of Figs. 1-3, which is inserted (block 102) into a cardiac chamber of the living subject; and/or from body surface electrodes 30 (Fig. 1) applied to the skin of the living subject; and noise picked up in the recording apparatus cable 84 from the noise 88 in the EP laboratory, as described in more detail below. For example, one or more of the electrodes 49 (Fig. 3) of the catheter 14 are in contact with the tissue (e.g., endocardial surface 53 (Fig. 3) of the chamber of the heart 12 (Fig. 1) and provide cardiac signal segments used to train the artificial neural network 75. In order to provide high quality training data, the operator 16 generally confirms that there is a good quality of contact between the tissue of the living subject and the electrode(s) 49 (and/or body surface electrodes 30) providing the cardiac signal segments.

The processing circuitry 22 is configured to receive (block 104) cardiac signal segments responsively to electrical activity sensed by one or more of the electrodes 49 (and/or one or more of the body surface electrodes 30) in contact with tissue of the living subject. The catheter 14 may provide signal segments from different electrodes 49 while in a given position within the cardiac chamber and/or from one or more electrodes 49 while the catheter 14 is moved to different positions in the cardiac chamber. The cardiac signal segments may be provided from different cardiac chambers and even from different living subjects.

The processing circuitry 22 is configured to receive the cardiac signal segments from the sensing electrode(s) 49 of the catheter 14 and/or from the body surface electrodes 30 via the analog-to-digital converter 70 and optionally the digital signal filtering circuitry 72. The analog-to-digital converter 70 is configured to convert (block 106) the received cardiac signal segments from analog form to digital form.

In some embodiments, the digital signal filtering circuitry 72 is coupled to receive the cardiac signal segments (now in digital form) from one or more of the sensing electrodes 49 and/or the body surface electrodes 30 and is configured to filter noise from the received signals or signal segments. The digital signal filtering circuitry 72 may include various filtering circuits, for example, but not limited to, a low pass filter to remove signals with frequencies higher than a threshold frequency (for example 60 Hertz or 100 Hertz), and/or a band-rejection filter to remove signals with frequencies in a range of frequencies (for example, from 100-200 Hz). The cardiac signals may include similar frequencies to noise, for example, in the 50 Hz range and therefore simply filtering out 50 Hz components using a low pass or band-rejection filter may not yield acceptable results. Therefore, other filtering methods may also be applied to remove noise associated with outside sources without adversely affecting the cardiac signals. At least some of the functionality of the digital signal filtering circuitry 72 may optionally be performed by one or more computers or processors executing software.

In some embodiments, the processing circuitry 22 generally receives continuous signals from the sensing electrodes 49 and the body surface electrodes 30. When the neural network training circuitry 76 is being trained, the training is performed using discrete signal segments as described in more detail below. Therefore, received signal(s) may be segmented logically, either using a synchronization signal generated by the synchronization circuitry 74 or using cross-correlation (in the neural network training circuitry 76) described in more detail below. In some embodiments, the signals may be segmented physically by the synchronization circuitry 74. In other embodiments, the synchronization circuitry 74 may segment the received cardiac signals by adding markers to the cardiac signals in order to identify the segments used as training data.

The processing circuitry 22 is configured to inject the cardiac signal segments (received via the analog-to-digital converter 70 and optionally via the digital signal filtering circuitry 72) into an end of the recording apparatus cable 84 via the digital-to-analog converter 80, which is configured to convert (block 108) the received cardiac signal segments from a digital form to an analog form. Therefore, the processing circuitry 22 is configured to inject (block 110) the received cardiac signal segments in analog form into one or more of the calibration wires at one end of the recording apparatus cable 84, which is configured to output corresponding noise-added cardiac signal segments responsively to electrical noise acquired in the recording apparatus cable 84 into the end of the shielded cable 90 closest to the recording apparatus 86. For example, noise is added to a cardiac signal segment A yielding a cardiac signal segment A', and noise is added to a cardiac signal segment B yielding a cardiac signal segment B'. The end of the shielded cable 90 closest to the processing circuitry 22 is configured to output the noise-added cardiac signal segments into the analog-to-digital converter 82 of the processing circuitry 22. The processing circuitry 22 is also configured to inject the cardiac signal segments into the neural network training circuitry 76 via the synchronization circuitry 74.

The analog-to-digital converter 82 is configured to receive (block 112) the noise-added cardiac signal segments outputted from the shielded cable 90 and convert (block 114) the noise-added cardiac signal segments from analog to digital form.

The received cardiac signals and the noise-added cardiac signal segments are received by the neural network training circuitry 76. The neural network training circuitry 76 of the processing circuitry 22 is configured to train (block 116) the artificial neural network 75 (e.g., the autoencoder 77) to at least partially compensate for electrical noise that will be added to cardiac signals in the recording apparatus cable 84 responsively to the received cardiac signal segments in digital form and the corresponding noise-added cardiac signal segments output by the end of the shielded cable 90 (closest to the processing circuitry 22) and now in digital form. The step of block 116 is described in more detail with reference to Figs. 6-8.

The processing circuitry 22 trains the artificial neural network 75 based on using cardiac signal segments that correspond with the noise-added cardiac signal segments. If a continuous cardiac signal is input into neural network training circuitry 76 and the same continuous cardiac signal is input into the recording apparatus cable 84 yielding a continuous noise-added cardiac signal, corresponding segments of the signals need to be identified for use in the training process. As mentioned above, the synchronization circuitry 74 may generate a synchronization signal (e.g., including periodic pulses) which is injected into its own calibration wire in the recording apparatus cable 84 and the shielded cable 90 as well as being sent to the neural network training circuitry 76 so that the neural network training circuitry 76 may identify segments of the received cardiac signal and the noise-added cardiac signal based on the synchronization signal. Alternatively, the signals may be divided into separate physical segments by the synchronization circuitry 74 for sending to the neural network training circuitry 76 and injecting into the recording apparatus cable 84. Alternatively, the neural network training circuitry 76 may apply cross-correlation between the cardiac signal segments received from the digital signal filtering circuitry 72 and the noise-added cardiac signal segments received from the shielded cable 90 in order to correctly align the cardiac signal segments and the noise-added cardiac signal segments.

In some embodiments, the artificial neural network 75 may comprise an ANN used for training and a different ANN used in production (to provide noise compensated cardiac signals). The parameters (e.g., weights of the artificial neural network 75) may be copied intermittently from the training ANN to the production ANN. In other embodiments, a single ANN may be used. Training of the ANN 75 may take place during the medical procedure so that the accuracy of the ANN 75 is improved and also so that the ANN 75 reacts to new electrical noise created in the EP room during the medical procedure.

Reference is now made to Fig. 6, which is a schematic view of the artificial neural network 75 for use with the system 10 of Fig. 1.

A neural network is a network or circuit of neurons, or in a modern sense, an artificial neural network, composed of artificial neurons or nodes. The connections of the biological neuron are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

These artificial networks may be used for predictive modeling, adaptive control and applications and can be trained via a dataset. Self-learning resulting from experience can occur within networks, which can derive conclusions from a complex and seemingly unrelated set of information.

For completeness, a biological neural network is composed of a group or groups of chemically connected or functionally associated neurons. A single neuron may be connected to many other neurons and the total number of neurons and connections in a network may be extensive. Connections, called synapses, are usually formed from axons to dendrites, though dendrodendritic synapses and other connections are possible. Apart from the electrical signaling, there are other forms of signaling that arise from neurotransmitter diffusion.

Artificial intelligence, cognitive modeling, and neural networks are information processing paradigms inspired by the way biological neural systems process data. Artificial intelligence and cognitive modeling try to simulate some properties of biological neural networks. In the artificial intelligence field, artificial neural networks have been applied successfully to speech recognition, image analysis and adaptive control, in order to construct software agents (in computer and video games) or autonomous robots.

A neural network (NN), in the case of artificial neurons called artificial neural network (ANN) or simulated neural network (SNN), is an interconnected group of natural or artificial neurons that uses a mathematical or computational model for information processing based on a connectionistic approach to computation. In most cases an ANN is an adaptive system that changes its structure based on external or internal information that flows through the network. In more practical terms, neural networks are non-linear statistical data modeling or decision-making tools. They can be used to model complex relationships between inputs and outputs or to find patterns in data.

In some embodiments, as shown in Fig. 6, the artificial neural network 75 may include the autoencoder 77 including an encoder 92 and a decoder 94. In other embodiments, the artificial neural network 75 may comprise any suitable ANN. The artificial neural network 75 may comprise software executed by the processing circuitry 22 (Fig. 4) and/or hardware modules configured to perform the functions of the artificial neural network 75.

The encoder 92 includes an input layer 96 into which an input is received. The encoder then includes one or more hidden layers 97 which progressively compress the input to a code 98. The decoder 94 includes one or more hidden layers 99 which progressively decompress the code 98 up to an output layer 95 from which the output of the autoencoder 77 is provided. The autoencoder 77 includes weights between the layers of the autoencoder 77. The autoencoder 77 manipulates the data received at the input layer 96 according to the values of the various weights between the layers of the autoencoder 77.

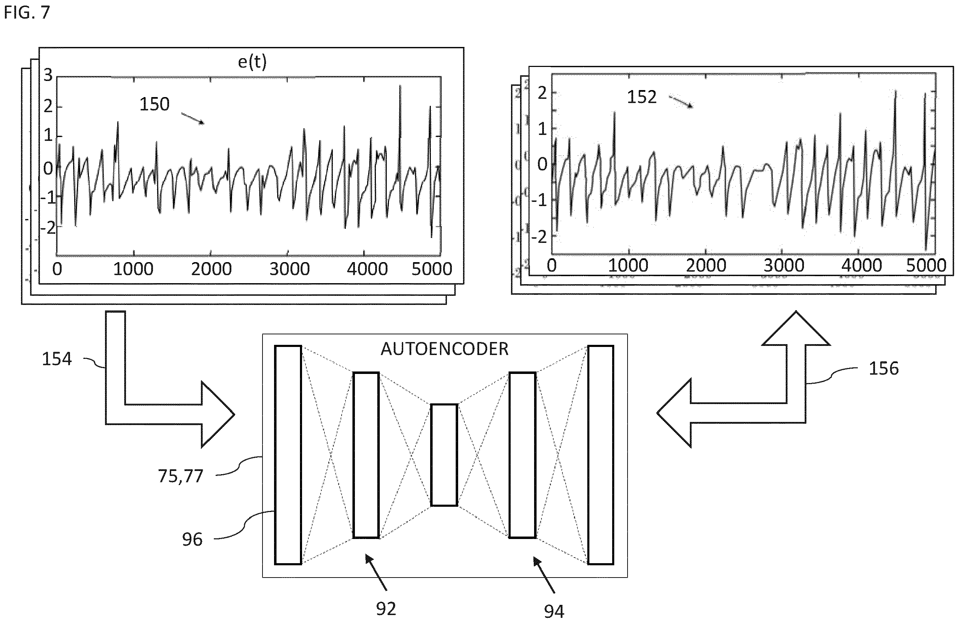

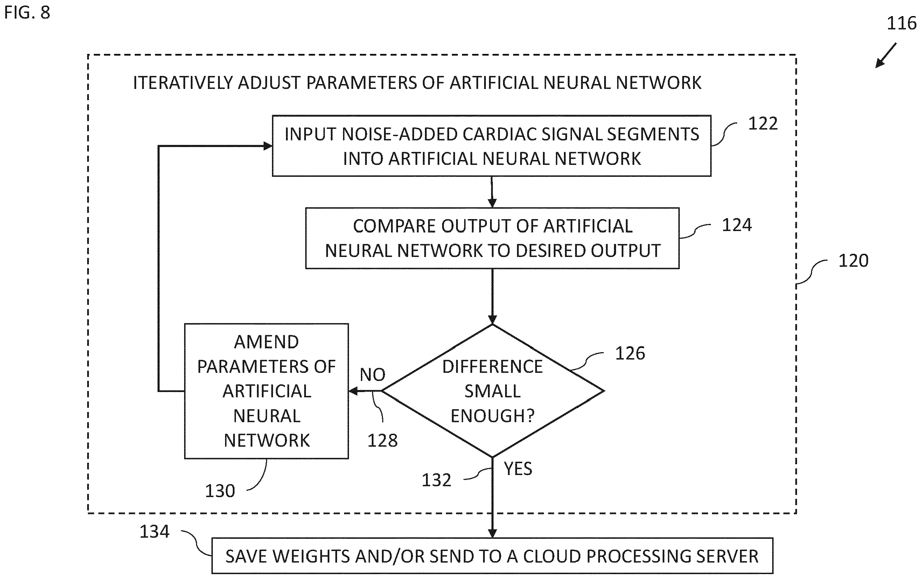

The weights of the autoencoder 77 are updated during training of the autoencoder 77 so that the autoencoder 77 performs a data manipulation task that the autoencoder 77 is trained to perform. In the example of Fig. 6, the autoencoder 77 is trained to remove future noise from cardiac signals as described in more detail with reference to Figs. 7 and 8.

The number of layers in the autoencoder 77 and the width of the layers may be configurable. As the number of layers and width of the layers increases so does the accuracy to which the autoencoder 77 can manipulate data according to the task at hand. However, a larger number of layers, and wider layers, generally requires more training data, more training time and the training may not converge. By way of example, the input layer 96 may include 400 neurons (e.g., to compress a batch of 400 samples). The encoder 92 may include five layers which compress by a factor of two (e.g., 400, 200, 100, 50, 25). The decoder 94 may include five layers which decompress by a factor of 2 (e.g., 25, 50, 100, 200, 400).

Reference is now made to Figs. 7 and 8. Fig. 7 is a schematic view illustrating training of the artificial neural network 75 of Fig. 6. Fig. 8 is a flowchart including sub-steps in the step of block 116 of Fig. 5.

The neural network training circuitry 76 of the processing circuitry 22 (Fig. 4) is configured to train the artificial neural network 75 (e.g., the autoencoder 77) to at least partially compensate for electrical noise that will be added to cardiac signals in the cable 84 (Fig. 4) responsively to the received cardiac signal segments (graph 152) and the corresponding noise-added cardiac signal segments (graph 150).

Training the artificial neural network 75 is generally an iterative process. One method of training the artificial neural network 75 is now described below. The neural network training circuitry 76 of the processing circuitry 22 (Fig. 4) is configured to iteratively adjust (block 120) parameters of the artificial neural network 75 to reduce a difference between an output of the artificial neural network 75 and a desired output (e.g., the received cardiac signal segments).

Sub-steps of the step of block 120 are now described below.

The neural network training circuitry 76 of the processing circuitry 22 (Fig. 4) is configured to input (block 122, arrow 154) the noise-added cardiac signal segments (graphs 150) into the artificial neural network 75. For example, the noise-added cardiac signals are input into the input layer 96 of the encoder 92. The neural network training circuitry 76 of the processing circuitry 22 (Fig. 4) is configured to compare (block 124, arrow 156) the output of the artificial neural network 75 (e.g., the output of the decoder 94 of the autoencoder 77) with the desired output, i.e., the corresponding received cardiac signal segments (graphs 152). For example, if there is a set of intracardiac signal segments A, B, C output by the artificial neural network 75 and a corresponding set of received intracardiac signal segments A', B', and C', the neural network training circuitry 76 of the processing circuitry 22 (Fig. 4) compares A with A', B with B', C with C' and so on. The comparison is generally performed using a suitable loss function, which computes the overall difference between all the outputs of the artificial neural network 75 and all the desired outputs (e.g., all the corresponding received intracardiac signal segments (graphs 152)).

At a decision block 126, the neural network training circuitry 76 of the processing circuitry 22 (Fig. 4) is configured to determine if the difference between the output of the artificial neural network 75 and desired output is small enough. If the difference between the output of the artificial neural network 75 and the desired output is small enough (branch 132), the neural network training circuitry 76 of the processing circuitry 22 (Fig. 4) is configured to save (block 134) the parameters (e.g., weights) of the artificial neural network 75 (e.g., the autoencoder 77) and/or send the parameters (e.g., weights) to a cloud processing server (not shown).

If the difference is not small enough (branch 128), the neural network training circuitry 76 of the processing circuitry 22 (Fig. 4) is configured to amend (block 130) parameters (e.g., weights) of the artificial neural network 75 (e.g., of the autoencoder 77) to reduce the difference between the output of the artificial neural network 75 and the desired output of the artificial neural network 75. The difference being minimized in the above example is the overall difference between all the outputs of the artificial neural network 75 and all the desired outputs (e.g., all the received intracardiac signal segments (graphs 152)). The neural network training circuitry 76 of the processing circuitry 22 (Fig. 4) is configured to amend the parameters using any suitable optimization algorithm, for example, a gradient descent algorithm such as Adam Optimization. The steps of blocks 122-126 are then repeated.

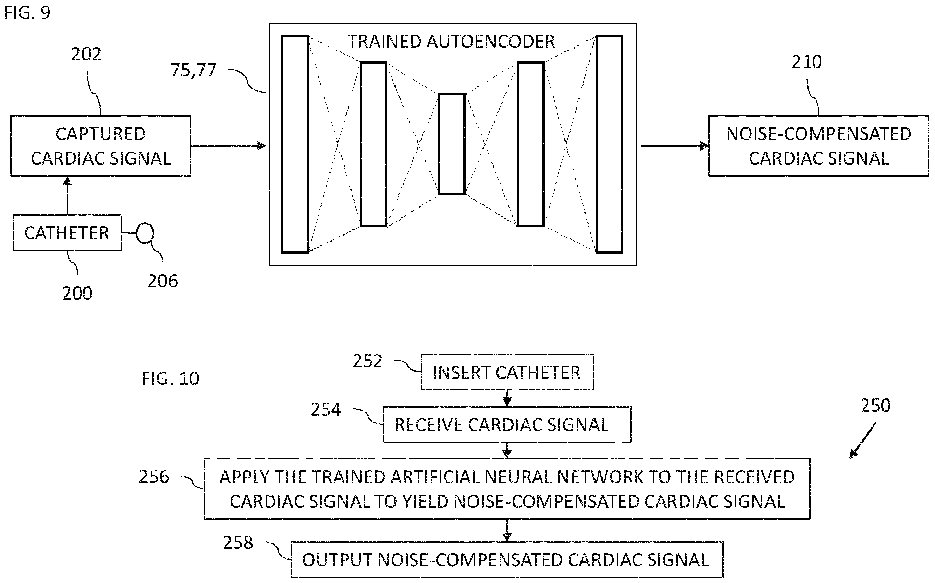

Reference is now made to Figs 9 and 10. Fig. 9 is a schematic view illustrating processing of a captured cardiac signal 202 being processed by the trained artificial neural network 75. Fig. 10 is a flowchart 250 including steps in a method to process the captured signal of Fig. 9 using the trained artificial neural network 75.

A catheter 200 is configured to be inserted into a cardiac chamber of a living subject. The catheter 200 includes one or more sensing electrodes 206 being configured to contact tissue of a living subject. The living subject may be: the same living subject into which the catheter 14 (Fig. 1) was inserted and according to which the artificial neural network 75 was trained; or a different living subject.

The medical system 10 is configured to yield a noise-compensated cardiac signal from the cardiac signal 202 using the trained artificial neural network 75 (Fig. 7) as described in more detail below.

The catheter 200 is an example of a catheter which provides a cardiac signal in which noise-compensation is added. Any suitable catheter (e.g., a balloon, basket, or focal catheter), may provide a cardiac signal which is then processed using the trained artificial neural network 75 to yield a noise-compensated cardiac signal. In some embodiments, the catheter 14 may be used to provide cardiac signals to be processed by the artificial neural network 75 to provide noise-compensated cardiac signals. In other words, the same catheter which trains the artificial neural network 75 may provide cardiac signals to be processed by the trained artificial neural network 75 to provide noise-compensated cardiac signals. In some embodiments, the same catheter provides cardiac signal segments to be used as training data to train the artificial neural network 75 at the same time as providing cardiac signals to be processed to yield noise-compensated cardiac signals from the trained artificial neural network 75, which is continually being trained using the training data.

Similarly, one or more cardiac signals provided by one or more body surface electrodes 30 (Fig. 1) may be processed by the artificial neural network 75 to add noise-compensation to the provided cardiac signal(s).

The catheter 200 is inserted (block 252) in a cardiac chamber of a living subject and/or body surface electrodes 30 are applied to a skin surface of the living subject.

The noise-compensation circuitry 78 of the processing circuitry 22 (Fig. 4) is configured to receive (block 254) the cardiac signal 202 responsively to electrical activity sensed by one of the sensing electrodes 206 (and/or the body surface electrodes 30) in contact with tissue of the living subject (e.g., while the catheter 200 is inserted into the cardiac chamber of the living subject). The cardiac signal 202 is received via the analog-to-digital converter 70 (Fig. 4), which is configured to convert the cardiac signal 202 from analog to digital form. The digital signal filtering circuitry 72 is optionally configured to remove noise from the cardiac signal 202.

The noise-compensation circuitry 78 of the processing circuitry 22 is configured to apply (block 256) the trained artificial neural network 75 to the cardiac signal 202 yielding the cardiac signal with noise-compensation 210, which at least partially compensates for the electrical noise, which is not yet in the cardiac signal 202 but will be added to the cardiac signal 202 in the cable 84 (Fig. 4).

In some embodiments, the trained artificial neural network comprises the trained autoencoder 77. In these embodiments, the noise-compensation circuitry 78 of the processing circuitry 22 is configured to apply the trained autoencoder 77 to the cardiac signal 202 yielding the cardiac signal with noise-compensation 210, which at least partially compensates for the electrical noise, which is not yet in the cardiac signal 202 but will be added to the cardiac signal 202 in the cable 84 (Fig. 4).

The processing circuitry 22 is configured to output (block 258) the cardiac signal with the noise-compensation 210 to the recording apparatus 86 (Fig. 4) via the digital-to-analog converter 80 (which is configured to convert the cardiac signal 202 with the noise-compensation 210 to analog form) and via the cable 84.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The present invention is not limited by what has been particularly shown and described hereinabove. Rather the scope of the invention is defined by the claims.

## Claims

1. A medical system (10), comprising:
a first sensing electrode (30, 49) configured to contact tissue of a first living subject;
a second sensing electrode (30, 49) configured to contact tissue of a second living subject;
a recording apparatus (86);
processing circuitry (22); and
a recording apparatus cable (84) extending between the recording apparatus and the processing circuitry;
wherein the processing circuitry is configured to:
receive first cardiac signal segments responsively to electrical activity sensed by the first sensing electrode in contact with the tissue;
inject the received first cardiac signal segments into the recording apparatus cable, the cable being configured to output corresponding noise-added cardiac signal segments responsively to electrical noise acquired in the cable;
receive the noise-added cardiac signal segments;
train an artificial neural network (75) to at least partially compensate for electrical noise that will be added to cardiac signals in the cable responsively to the received first cardiac signal segments and the corresponding noise-added cardiac signal segments;
receive a second cardiac signal responsively to electrical activity sensed by the second sensing electrode in contact with the tissue of the second living subject;
apply the trained artificial neural network to the second cardiac signal yielding the second cardiac signal with noise-compensation, which at least partially compensates for the electrical noise, which is not yet in the second cardiac signal but will be added to the second cardiac signal in the cable; and
output the second cardiac signal with the noise-compensation to the recording apparatus via the cable.

2. The system (10) according to claim 1, wherein the processing circuitry (22) is configured to:
input the noise-added cardiac signal segments into the artificial neural network (75); and
iteratively adjust parameters of the artificial neural network to reduce a difference between an output of the artificial neural network and the received first cardiac signal segments.

3. The system (10) according to claim 1, wherein the processing circuitry (22) further comprises:
a digital-to-analog converter (80) configured to convert the first cardiac signal segments from a digital form to an analog form, the processing circuitry being configured to inject the first cardiac signal segments in the analog form into the cable (84); and
an analog-to-digital converter (82) configured to convert the noise-added cardiac signal segments to digital form, the processing circuitry being configured to train the artificial neural network (75) to at least partially compensate for electrical noise that will be added to cardiac signals in the cable responsively to the received first cardiac signal segments in digital form and the corresponding noise-added cardiac signal segments in digital form.

4. The system (10) according to claim 1, wherein the artificial neural network (75) comprises an autoencoder (77) including an encoder (92) and a decoder (94), the processing circuitry being configured to train the autoencoder to at least partially compensate for electrical noise that will be added to cardiac signals in the cable (84) responsively to the received first cardiac signal segments and the corresponding noise-added cardiac signal segments.

5. The system (10) according to claim 4, wherein the processing circuitry (22) is configured to apply the autoencoder (77) to the second cardiac signal yielding the second cardiac signal with the noise-compensation.

6. The system (10) according to claim 1, further comprising:
a first catheter (14) comprising the first sensing electrode (49), and configured to be inserted into a cardiac chamber of the first living subject; and
a second catheter (200, 14) comprising the second sensing electrode, and configured to be inserted into a cardiac chamber of the second living subject.

7. The system (10) according to claim 6, wherein the first catheter (14) includes the second catheter.

8. The system (10) according to claim 1, further comprising a shielded cable (90) having a first end and a second end, wherein:
the recording apparatus cable (84) has a first end electrically connected to the processing circuitry (22) and a second end electrically connected to the recording apparatus (86) and the first end of the shielded cable;
the second end of the shielded cable is electrically connected to the second end of the recording apparatus cable;
the processing circuitry is configured to inject the received first cardiac signal segments into the first end of the recording apparatus cable;
the second end of the shielded cable is configured to output the noise-added cardiac signal segments; and
the processing circuitry is configured to train the artificial neural network (75) responsively to the received first cardiac signal segments and the corresponding noise-added cardiac signal segments output by the second end of the shielded cable.

## Patentansprüche

1. Medizinisches System (10), umfassend:
eine erste Sensorelektrode (30, 49), die dafür ausgelegt ist, Gewebe eines ersten lebenden Subjekts zu kontaktieren;
eine zweite Sensorelektrode (30, 49), die dafür ausgelegt ist, Gewebe eines zweiten lebenden Subjekts zu kontaktieren;
eine Aufzeichnungsvorrichtung (86);
Verarbeitungsschaltungen (22); und
ein Aufzeichnungsvorrichtungskabel (84), das sich zwischen der Aufzeichnungsvorrichtung und den Verarbeitungsschaltungen erstreckt;
wobei die Verarbeitungsschaltungen ausgelegt sind zum:
Empfangen erster Herzsignalsegmente in Reaktion auf elektrische Aktivität, die von der ersten Sensorelektrode erkannt wird, welche in Kontakt mit dem Gewebe steht;
Einspeisen der empfangenen ersten Herzsignalsegmente in das Aufzeichnungsvorrichtungskabel, wobei das Kabel dafür ausgelegt ist, in Reaktion auf das Beziehen von elektrischem Rauschen in dem Kabel entsprechende rauschbehaftete Herzsignalsegmente auszugeben;
Empfangen der rauschbehafteten Herzsignalsegmente;
Trainieren eines künstlichen neuronalen Netzes (75), elektrisches Rauschen, das Herzsignalen in dem Kabel in Reaktion auf die empfangenen ersten Herzsignalsegmente und die entsprechenden rauschbehafteten Herzsignalsegmente hinzugefügt wird, wenigstens teilweise zu kompensieren;
Empfangen eines zweiten Herzsignals in Reaktion auf elektrische Aktivität, die von der zweiten Sensorelektrode erkannt wird, welche in Kontakt mit dem Gewebe des zweiten lebenden Subjekts steht;
Anwenden des trainierten künstlichen neuronalen Netzes auf das zweite Herzsignal, was das zweite Herzsignal mit Rauschkompensation ergibt, wodurch das elektrische Rauschen, das noch nicht in dem zweiten Herzsignal vorhanden ist, aber dem zweiten Herzsignal in dem Kabel hinzugefügt wird, wenigstens teilweise kompensiert wird; und
Ausgeben des zweiten Herzsignals mit der Rauschkompensation an die Aufzeichnungsvorrichtung über das Kabel.

2. System (10) gemäß Anspruch 1, wobei die Verarbeitungsschaltung (22) ausgelegt ist zum:
Eingeben der rauschbehafteten Herzsignalsegmente in das künstliche neuronale Netz (75); und
iteratives Anpassen von Parametern des künstlichen neuronalen Netzes, um eine Differenz zwischen einer Ausgabe des künstlichen neuronalen Netzes und den empfangenen ersten Herzsignalsegmenten zu reduzieren.

3. System (10) gemäß Anspruch 1, wobei die Verarbeitungsschaltung (22) ferner umfasst:
einen Digital/Analog-Wandler (80), der dafür ausgelegt ist, die ersten Herzsignalsegmente von einer digitalen Form in eine analoge Form umzuwandeln, wobei die Verarbeitungsschaltungen dafür ausgelegt sind, die ersten Herzsignalsegmente in analoger Form in das Kabel (84) einzuspeisen; und
einen Analog/Digital-Wandler (82), der dafür ausgelegt ist, die rauschbehafteten Herzsignalsegmente in eine digitale Form umzuwandeln, wobei die Verarbeitungsschaltungen dafür ausgelegt sind, das künstliche neuronale Netz (75) dafür zu trainieren, elektrisches Rauschen, das den Herzsignalen in dem Kabel in Reaktion auf die empfangenen ersten Herzsignalsegmente in digitaler Form und die entsprechenden rauschbehafteten Herzsignalsegmente in digitaler Form hinzugefügt wird, wenigstens teilweise zu kompensieren.

4. System (10) gemäß Anspruch 1, wobei das künstliche neuronale Netz (75) einen Autocodierer (77) umfasst, der einen Codierer (92) und einen Decodierer (94) aufweist, wobei die Verarbeitungsschaltungen dafür ausgelegt sind, den Autocodierer dafür zu trainieren, elektrisches Rauschen, das den Herzsignalen in dem Kabel (84) in Reaktion auf die empfangenen ersten Herzsignalsegmente und die entsprechenden rauschbehafteten Herzsignalsegmente hinzugefügt wird, wenigstens teilweise zu kompensieren.

5. System (10) gemäß Anspruch 4, wobei die Verarbeitungsschaltungen (22) dafür ausgelegt sind, den Autocodierer (77) auf das zweite Herzsignal anzuwenden, was das zweite Herzsignal mit der Rauschkompensation ergibt.

6. System (10) gemäß Anspruch 1, ferner umfassend:
einen ersten Katheter (14), umfassend die erste Sensorelektrode (49) und dafür ausgelegt, in eine Herzkammer des ersten lebenden Subjekts eingeführt zu werden; und
einen zweiten Katheter (200), umfassend die zweite Sensorelektrode (14) und dafür ausgelegt, in eine Herzkammer des zweiten lebenden Subjekts eingeführt zu werden.

7. System (10) gemäß Anspruch 6, wobei der erste Katheter (14) den zweiten Katheter beinhaltet.

8. System (10) gemäß Anspruch 1, ferner umfassend ein abgeschirmtes Kabel (90) mit einem ersten Ende und einem zweiten Ende, wobei:
das Aufzeichnungsvorrichtungskabel (84) ein erstes Ende aufweist, das elektrisch mit den Verarbeitungsschaltungen (22) verbunden ist, und ein zweites Ende, das elektrisch mit der Aufzeichnungsvorrichtung (86) und dem ersten Ende des abgeschirmten Kabels verbunden ist;
das zweite Ende des abgeschirmten Kabels elektrisch mit dem zweiten Ende des Aufzeichnungsvorrichtungskabels verbunden ist;
die Verarbeitungsschaltungen dafür ausgelegt sind, die empfangenen ersten Herzsignalsegmente in das erste Ende des Aufzeichnungsvorrichtungskabels einzuspeisen;
das zweite Ende des abgeschirmten Kabels dafür ausgelegt ist, die rauschbehafteten Herzsignalsegmente auszugeben; und
die Verarbeitungsschaltungen dafür ausgelegt sind, das künstliche neuronale Netz (75) in Reaktion auf die empfangenen ersten Herzsignalsegmente und die entsprechenden rauschbehafteten Herzsignalsegmente, die von dem zweiten Ende des abgeschirmten Kabels ausgegeben werden, zu trainieren.

## Revendications

1. Système médical (10), comprenant :
une première électrode de détection (30, 49) configurée pour entrer en contact avec du tissu d'un premier sujet vivant ;
une deuxième électrode de détection (30, 49) configurée pour entrer en contact avec du tissu d'un deuxième sujet vivant ;
un appareil d'enregistrement (86) ;
un circuit de traitement (22) ; et
un câble d'appareil d'enregistrement (84) s'étendant entre l'appareil d'enregistrement et le circuit de traitement ;
dans lequel le circuit de traitement est configuré pour :
recevoir de premiers segments de signal cardiaque en réponse à de l'activité électrique détectée par la première électrode de détection en contact avec le tissu ;
injecter les premiers segments de signal cardiaque reçus dans le câble d'appareil d'enregistrement, le câble étant configuré pour délivrer des segments de signal cardiaque avec bruit ajouté correspondants en réponse à du bruit électrique acquis dans le câble ;
recevoir les segments de signal cardiaque avec bruit ajouté ;
entraîner un réseau de neurones artificiels (75) à compenser au moins partiellement du bruit électrique qui sera ajouté à des signaux cardiaques dans le câble en réponse aux premiers segments de signal cardiaque reçus et aux segments de signal cardiaque avec bruit ajouté correspondants ;
recevoir un deuxième signal cardiaque en réponse à de l'activité électrique détectée par la deuxième électrode de détection en contact avec le tissu du deuxième sujet vivant ;
appliquer le réseau de neurones artificiels entraîné au deuxième signal cardiaque, ce qui produit le deuxième signal cardiaque avec compensation du bruit, qui compense au moins partiellement le bruit électrique qui n'est pas encore dans le deuxième signal cardiaque, mais sera ajouté au deuxième signal cardiaque dans le câble ; et
délivrer le deuxième signal cardiaque avec la compensation du bruit à l'appareil d'enregistrement par le biais du câble.

2. Système (10) selon la revendication 1, dans lequel le circuit de traitement (22) est configuré pour :
entrer les segments de signal cardiaque avec bruit ajouté dans le réseau de neurones artificiels (75) ; et
ajuster par itérations des paramètres du réseau de neurones artificiels pour réduire une différence entre une sortie du réseau de neurones artificiels et les premiers segments de signal cardiaque reçus.

3. Système (10) selon la revendication 1, dans lequel le circuit de traitement (22) comprend en outre :
un convertisseur numérique-analogique (80) configuré pour convertir les premiers segments de signal cardiaque d'une forme numérique à une forme analogique, le circuit de traitement étant configuré pour injecter les premiers segments de signal cardiaque sous la forme analogique dans le câble (84) ; et
un convertisseur analogique-numérique (82) configuré pour convertir les segments de signal cardiaque avec bruit ajouté en forme numérique, le circuit de traitement étant configuré pour entraîner le réseau de neurones artificiels (75) à compenser au moins partiellement du bruit électrique qui sera ajouté à des signaux cardiaques dans le câble en réponse aux premiers segments de signal cardiaque reçus sous forme numérique et aux segments de signal cardiaque avec bruit ajouté correspondants sous forme numérique.

4. Système (10) selon la revendication 1, dans lequel le réseau de neurones artificiels (75) comprend un auto-encodeur (77) comportant un encodeur (92) et un décodeur (94), le circuit de traitement étant configuré pour entraîner l'auto-encodeur à compenser au moins partiellement du bruit électrique qui sera ajouté à des signaux cardiaques dans le câble (84) en réponse aux premiers segments de signal cardiaque reçus et aux segments de signal cardiaque avec bruit ajouté correspondants.

5. Système (10) selon la revendication 4, dans lequel le circuit de traitement (22) est configuré pour appliquer l'auto-encodeur (77) au deuxième signal cardiaque, ce qui produit le deuxième signal cardiaque avec la compensation du bruit.

6. Système (10) selon la revendication 1, comprenant en outre :
un premier cathéter (14) comprenant la première électrode de détection (49), et configuré pour être inséré dans une chambre cardiaque du premier sujet vivant ; et
un deuxième cathéter (200, 14) comprenant la deuxième électrode de détection, et configuré pour être inséré dans une chambre cardiaque du deuxième sujet vivant.

7. Système (10) selon la revendication 6, dans lequel le premier cathéter (14) inclut le deuxième cathéter.

8. Système (10) selon la revendication 1, comprenant en outre un câble blindé (90) ayant une première extrémité et une deuxième extrémité, dans lequel :
le câble d'appareil d'enregistrement (84) a une première extrémité reliée électriquement au circuit de traitement (22) et une deuxième extrémité reliée électriquement à l'appareil d'enregistrement (86) et à la première extrémité du câble blindé ;
la deuxième extrémité du câble blindé est reliée électriquement à la deuxième extrémité du câble d'appareil d'enregistrement ;
le circuit de traitement est configuré pour injecter les premiers segments de signal cardiaque reçus dans la première extrémité du câble d'appareil d'enregistrement ;
la deuxième extrémité du câble blindé est configurée pour délivrer les segments de signal cardiaque avec bruit ajouté ; et
le circuit de traitement est configuré pour entraîner le réseau de neurones artificiels (75) en réponse aux premiers segments de signal cardiaque reçus et aux segments de signal cardiaque avec bruit ajouté correspondants délivrés par la deuxième extrémité du câble blindé.
